# EUROPÄISCHE PATENTSCHRIFT

(11) **EP 3 010 562 B1**
(45) Veröffentlichungstag und Bekanntmachung des Hinweises auf die Patenterteilung: **31.10.2018**
(21) Anmeldenummer: 14742141.6
(22) Anmeldetag: 13.06.2014
(51) Int. Cl.: A61M 1/10, A61M 1/12

(54) **HERZUNTERSTÜTZUNGSSYSTEM**
CARDIAC SUPPORT SYSTEM
SYSTÈME D'ASSISTANCE CARDIAQUE

(30) Priorität: 18.06.2013 DE 102013106352
(43) Veröffentlichungstag der Anmeldung: 27.04.2016
(73) Patentinhaber: Universität zu Lübeck, 23562 Lübeck (DE)
(72) Erfinder: SIEVERS, Hans-Hinrich, 24119 Kronshagen (DE)
(74) Vertreter: Hansen, Jochen
(86) Internationale Anmeldenummer: PCT/DE2014/100197
(87) Internationale Veröffentlichungsnummer: WO 2014/202051

(56) Entgegenhaltungen:
- FR-A1- 2 485 928
- US-A- 4 994 078
- US-A1- 2002 128 587
- US-A1- 2004 054 251

## Beschreibung

Die Erfindung betrifft ein Herzunterstützungssystem umfassend einen Ansaugbereich zur Ansaugung von Blut, einen Auslassbereich zur Ausgabe des angesaugten Blutes, eine Pumpanordnung zum Transport des Blutes vom Ansaugbereich zum Auslassbereich, eine Pumpen-Aorta-Verbindung und eine Saugverbindung vom Ansaugbereich zur Pumpe, wobei Teile der Herzfunktion durch das Herzunterstützungssystem übernommen werden.

Ferner betrifft die Erfindung ein Herzunterstützungsverfahren, insbesondere mit einem erfindungsgemäßen Herzunterstützungssystem, umfassend die Schritte Ansaugen von Blut innerhalb der linken Herzhälfte, Pumpen und Weiterleiten des angesaugten Blutes in die Aorta.

Eine Herzinsuffizienz ist eine krankhafte Unfähigkeit des Herzens, die vom Körper benötigte Blutmenge ohne Druckanstieg in den Herzvorhöfen zu fördern. Insbesondere führen Herzkammern, die vergrößert/dilatiert sind und damit keine ausreichende Leistung zum Bluttransport mehr erreichen können, zu dieser Herzinsuffizienz.

Eine medikamentöse Behandlung einer derartigen Herzinsuffizienz ist in der Regel nach einer gewissen Zeit ausgereizt bzw. austherapiert, wodurch letztendlich die Herzinsuffizienz mittels eines Herzunterstützungssystems versorgt werden muss, wobei dieses Herzunterstützungssystem die Bluttransportleistung wieder herstellen soll.

Aus dem Stand der Technik ist bekannt, ein Herzunterstützungssystem über die Herzspitze mittels eines daumengroßen Einflussstutzens zu implantieren, wobei dieser Einflussstutzen ca. 3 cm tief innerhalb des linken Ventrikels angeordnet wird. Hierbei bildet der Einflussstutzen eine Kanüle aus.

Aus der Druckschrift DE 695 31 399 T2 ist ein Herzunterstützungssystem in Form eines Kunstherzens bekannt, wobei eine Pumpanordnung innerhalb der linken Herzkammer angeordnet ist und Blut aus der linken Herzkammer durch die Aortenklappe hindurch in die Aorta leitet.

Die DE 697 30 617 T2 zeigt ein Kreislaufhilfssystem, welches die Pumpfunktion des Herzens eines Patienten unterstützt oder vorübergehend ersetzt mit einer außerhalb des Körpers liegenden, tragbaren Blutflußpumpe, die über eine prozessorgesteuerte Steuereinheit die Herzfunktion regelt.

Die US 8 226 712 B1 und US 6 048 363 A zeigen ein künstliches Herz mit automatischer Blutfluß- und -druckregelung, ein sogenanntes "TAH system" (Total Artificial Heart) bei dem die Funktion des Herzens übernommen wird bzw. ein sogenanntes LVAD (Left Ventricle Assist Device), bei dem eine Pumpe direkt am linken Vorhof angeschlossen wird und dabei ein Teil, u.a. die Mitralklappe, des Herzens umgangen wird.

Die US 4 994 078 A zeigt einige Bauarten eines künstlichen Herzens mit Pumpen die keine Klappen benötigen und ein Herzunterstützungssystem, das in der Herzkammer eingebracht ist und als hydraulischer Sack durch Muskelkraft angeregt wird, um die Funktion der Herzkammer zu ermöglichen.

Problematisch am derzeitigen Stand der Technik der Einflussstutzen/Kanülen ist, dass sich Thromben an der Kanüle selbst als auch hinter der Mitralklappe entwickeln. Die Thrombenbildung ist bedingt durch die mechanische Entlastung der linken Herzkammer, wodurch der Fluss in der linken Herzkammer quasi auf null reduziert wird.

Die Gabe von Medikamenten zur Hemmung der Blutgerinnung, Antikoagulanzien, kann nicht die Entstehung derartiger Gerinnungsthromben verhindern, so dass die sich bildenden Gerinnungsthromben in den gesamten Körper des Patienten verschleppt werden können, die dann nicht selbstständig vom Körper aufgelöst werden können und letztendlich zu großen Problemen für den Patienten führen können.

Der vorliegenden Erfindung liegt die Aufgabe zugrunde, eine Herzunterstützungssystem sowie ein Herzunterstützungsverfahren aufzuzeigen, die es ermöglichen, das Problem der Thrombosebildung im Bereich der linken Herzkammer bei Verwendung eines Herzunterstützungssystems zu verhindern, so dass die Gefahr einer Thrombenbildung durch das Herzunterstützungssystem vollständig ausgeschlossen wird.

Gelöst wird diese Aufgabe mit einem Herzunterstützungssystem nach Anspruch 1.

In einer ersten Alternative ist der Ansaugbereich direkt im Anschluss an die Mitralklappe angeordnet, wobei hierbei die natürliche Mitralklappe des Patienten erhalten bleibt und die Verbindung über eine spezielle Prothese kammerseitig an die Mitralklappe mittels Naht erfolgt.

In einer zweiten Alternative ist der Ansaugbereich als künstliche Mitralklappe ausgebildet und ersetzt eine einfache künstliche Mitralklappe oder die nicht mehr ausreichend funktionstüchtige natürliche Mitralklappe des Patienten.

Beide Alternativen haben gemein, dass das anzusaugende Blut ausschließlich aus dem linken Vorhof bestimmungsgemäß, was diesbezüglich bedeutet, dass das Herzunterstützungssystem in und an dem Herzen angeordnet ist, entnommen wird. Hierdurch wird das Blut aus dem linken Vorhof abgesaugt und durch den linken Ventrikel hindurchgeführt, so dass der linke Ventrikel praktisch ohne Funktion ist. Es können keine Thromben mehr in das Herzunterstützungssystem gelangen, wodurch sich eine deutliche Verbesserung der klinischen Problematik ergibt.

Entgegen der bisherigen Einschätzung, dass der linke Ventrikel sich immer erholen könnte, so dass das Herzunterstützungssystem unter Umständen wieder explantiert werden könnte, ist dies bei einem nicht mehr funktionstüchtigen "ausgebrannten" linken Ventrikel jedoch vollkommen ausgeschlossen. Im Vorwege kann bereits genau festgelegt werden, ob ein Ventrikel überhaupt noch die Möglichkeit haben kann, sich zu erholen oder ob alternativ die Indikation für das diesseitige erfindungsgemäße Herzunterstützungssystem sowie die Anwendung des erfindungsgemäßen Herzunterstützungsverfahrens angezeigt ist.

Die Saugverbindung besteht in der Variante, dass die natürliche Mitralklappe des Patienten ersetzt wird bzw. bereits eine künstliche Mitralklappe vorhanden ist, aus einem Antriebsstutzen der Pumpenanordnung und einem daran angeordneten mitral-apikalen Anschlussstück, wobei am pumpenfernen Ende eine künstliche Mitralklappe angeordnet ist, wobei die künstliche Mitralklappe im Bereich der natürlichen Mitralklappe der linken Herzkammer bestimmungsgemäß anordenbar ist.

Die Saugverbindung besteht alternativ bei noch gut erhaltener und funktionstüchtiger Mitralklappe aus einem Antriebsstutzen der Pumpenanordnung und einem daran angeordneten mitral-apikalen Anschlussstück, wobei am pumpenfernen Ende eine Mitralklappenringprothese angeordnet ist, wobei die Mitralklappenringprothese an dem Mitralklappenring bestimmungsgemäß von der linken Herzkammer anordenbar ist.

Der Ansaugbereich und die Saugverbindung sind innerhalb der linken Herzkammer/Ventrikel bestimmungsgemäß geführt. Die linke Herzkammer wird so quasi ohne Funktion geschaltet.

Die Pumpen-Aorta-Verbindung ist außerhalb der linken Herzkammer bestimmungsgemäß geführt, da auf diese Weise ein guter Blutfluss realisiert wird und die Pumpenanordnung entsprechend einfach ausgestaltet werden kann. Der Auslassbereich ist bestimmungsgemäß innerhalb der Aorta vorgesehen, so dass das aus dem linken Vorhof abgesaugte Blut direkt in die Aorta eingeleitet werden kann, wobei die Aortenklappe umgangen wird.

Bestimmungsgemäß kann eine zusätzliche Kanüle zwischen der linken Herzkammer und dem rechten Vorhof vorgesehen sein, falls ein Überdruck in der nunmehr außer Funktion gesetzten linken Herzkammer sich ausbilden sollte und dieser sich nicht selbst über die Aortenklappe bereinigt.

Die Saugverbindung weist zumindest abschnittsweise eine wenigstens auf der Innenseite spiralförmig ausgebildete Wandung auf, wobei ein in Pumpenrichtung wirkender unterstützender Bluttransport erfolgt. Durch diese besondere Ausgestaltung ist es möglich, den Bluttransport zu unterstützen.

Die spiralförmig ausgebildete Wandung der Saugverbindung ist mit einer geometrische Strukturierung, insbesondere durch Erhebungen, Bahnen, Leitkanäle, Leitwandungen, Einkerbungen und/oder Ausbuchtungen der Innenwand eine Beschleunigung des Blutes in Blutrichtung unterstützt.

Bevorzugt erfolgt durch die Ausgestaltung der Innenwandung der Saugverbindung mit einer spiralförmig in Richtung des Bluttransportes ausgebildeten Struktur eine Beschleunigung des Bluttransportes innerhalb der Saugverbindung.

Die Saugverbindung ist einteilig ausgebildet.

Die Saugverbindung weist eine Sensorik zur Erfassung der Strömungsgeschwindigkeit, Strömungsbeschleunigung und/oder des Blutdurchflusses aufweist, wobei die Sensorik mit der die Pumpanordnung zum Transport des Blutes verbindbar ist.

Die Pumpanordnung zum Transport des Blutes weist eine Regeleinheit auf, die mit der Sensorik verbindbar ist.

Das erfindungsgemäße Herzunterstützungsverfahren ist gekennzeichnet durch das Ansaugen des Blutes unmittelbar nach der Mitralklappe, wobei dieses derart erfolgt, dass das Blut aus dem linken Vorhof entnommen wird.

Der Bluttransport erfolgt ausschließlich durch das Absaugen des Blutes aus dem linken Vorhof unter Umgehung der linken Herzkammer und Einleiten des abgesaugten Blutes in die Aorta.

Ein sich bildender Überdruck innerhalb der linken Herzkammer kann in den rechten Vorhof abgeleitet werden.

Nachfolgend wird ein Ausführungsbeispiel der Erfindung anhand der beiliegenden Zeichnung detailliert beschrieben.

Darin zeigt:
- Fig. 1: eine schematische Darstellung eines ersten Ausführungsbeispiels des erfindungsgemäßen Herzunterstützungssystems;
- Fig. 2: eine schematische Darstellung eines zweiten Ausführungsbeispiels des erfindungsgemäßen Herzunterstützungssystem und
- Fig. 3: eine schematische Darstellung eines dritten Ausführungsbeispiels des erfindungsgemäßen Herzunterstützungssystems mit Regelungseinheit.

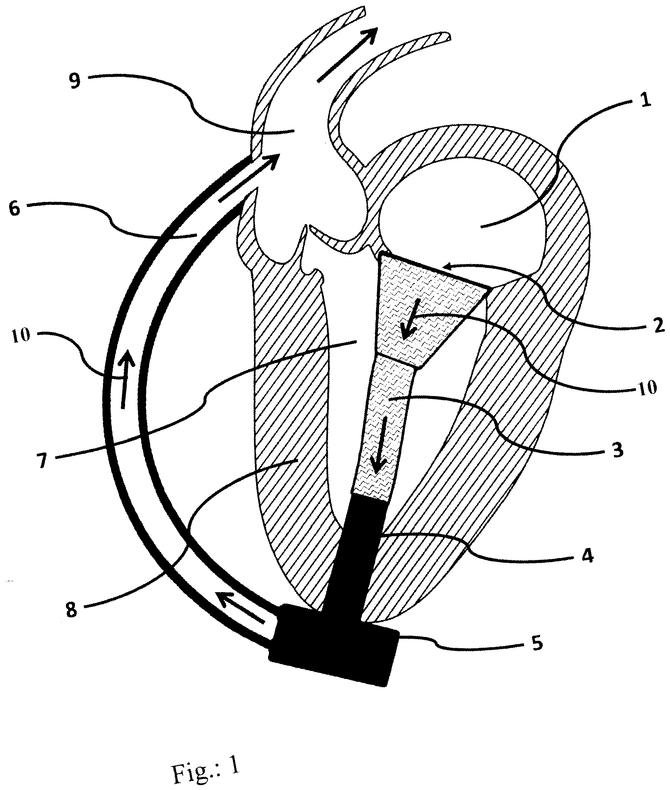

In Fig. 1 ist eine schematische Darstellung eines ersten Ausführungsbeispiels des erfindungsgemäßen Herzunterstützungssystems dargestellt.

Der dargestellte Teil des Herzens besteht aus dem linken Vorhof 1, dem linken Ventrikel / der linken Herzkammer 7, der Aorta 9 sowie der zwischen der linken Herzkammer 7 und der Aorta 9 angeordneten Aortenklappe und dem Mitralklappenbereich 2 zwischen dem linken Vorhof 1 und der linken Herzkammer 7.

Das erfindungsgemäße Herzunterstützungssystem umfasst eine Pumpe 5, eine Pumpen-Aorta-Verbindung 6, die die Pumpe mit der Aorta 9 verbindet, so dass Blut in die Aorta 9 in Blutrichtung 10 gefördert werden kann.

Weiter umfasst das erfindungsgemäße Herzunterstützungssystem an der Pumpe 5 ein Antriebsaggregatsstutzen, Saugverbindung 4, an dessen pumpenfernen Ende ein mitral-apikales Anschlussstück 3 angeordnet ist. An dem mitral-apikalen Anschlussstück 3 ist an dessen pumpenfernen Ende eine künstliche Mitralklappe 2 angeordnet.

Erfindungsgemäß wird nunmehr aus dem linken Vorhof 1 Blut in das mitralapikale Anschlussstück 3 durch die künstliche Mitralklappe 2 hindurch abgesaugt und in Blutrichtung 10 mit Hilfe der Pumpe 5 durch die Pumpen-Aorta-Verbindung 6 in die Aorta 9 gefördert. Hierbei wird die linke Herzkammer 7 vollständig umgangen.

Nachfolgend sind noch zwei weitere Ausführungsbeispiele erläutert, wobei zu der generellen Funktion der einzelnen Elemente auf die Figurenbeschreibung der Fig. 1 verwiesen wird.

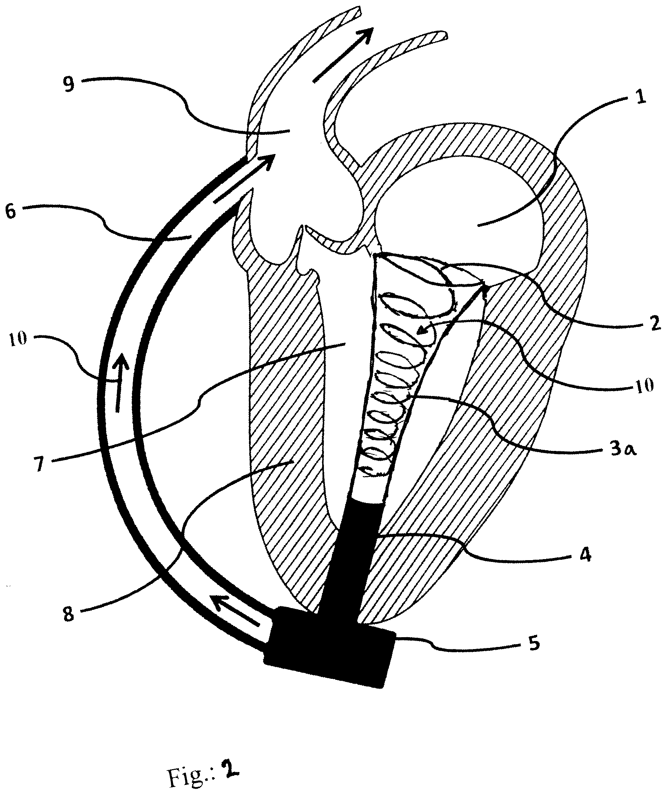

In Fig. 2 ist eine schematische Darstellung eines zweiten Ausführungsbeispiels des erfindungsgemäßen Herzunterstützungssystems dargestellt.

Die Saugverbindung 2, 3, 4 ist einteilig ausgeführt und auf der Innenwandung mit einer spiralförmigen Geometrie 3a, z.B. als Topographie, versehen, um den Blutfluss zu orientieren und gegebenenfalls zu beschleunigen.

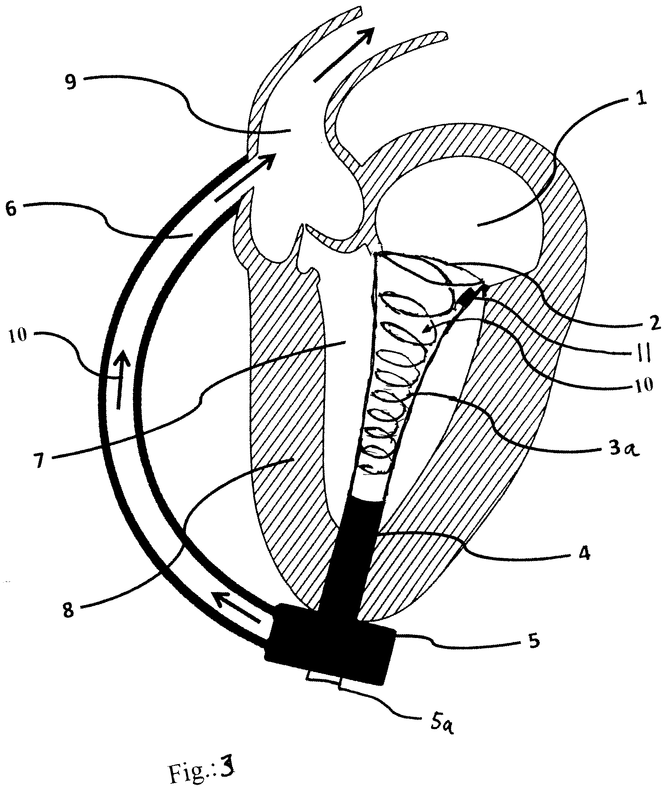

Fig. 3 zeigt eine schematische Darstellung eines dritten Ausführungsbeispiels des erfindungsgemäßen Herzunterstützungssystems mit Regelungseinheit.

Die Regelungseinheit 5a steuert die Pumpenanordnung 5 an, wobei hierzu die Signale der Sensorik 11 verwendet, erfasst, ausgewertet und gespeichert und/oder vorzugsweise auch gesendet werden, so dass die Pumpenanordnung 5 entsprechend des Blutflusses und/oder des Blutbedarfes individuell optimiert werden kann.

Die Energieversorgung der Pumpenanordnung sowie der weiteren aktiven Komponenten erfolgt bevorzugt autark, beispielsweise über Thermogeneratoren durch den Körper, Batterien und/oder körpernahe kabellose Energieübertragungsverfahren.

### Bezugszeichenliste

- 1: linker Vorhof
- 2: Mitralklappe
- 3: mitral-apikales Anschlussstück
- 3a: spiralförmige Innenwandung / Geometrie
- 4: Antriebsaggregatsstutzen, Saugverbindung
- 5: Pumpe
- 5a: Regeleinheit
- 6: Pumpen-Aorta-Verbindung
- 6a: Auslassbereich
- 7: linke Herzkammer
- 8: Herzkammerwand
- 9: Aorta
- 10: Blutrichtung
- 11: Sensorik

## Patentansprüche

1. Herzunterstützungssystem umfassend
- einen Ansaugbereich zur Ansaugung von Blut,
- einen Auslassbereich (6a) zur Ausgabe des angesaugten Blutes,
- eine Pumpanordnung (5) zum Transport des Blutes vom Ansaugbereich zum Auslassbereich (6a),
- eine Pumpen-Aorta-Verbindung (6), die außerhalb der linken Herzkammer (7) führbar ist, und
- eine Saugverbindung (4) vom Ansaugbereich zur Pumpe,
wobei Teile der Herzfunktion durch das Herzunterstützungssystem übernommen werden, wobei
der Ansaugbereich direkt im Anschluss an eine künstliche Mitralklappe (2) angeordnet ist
oder
der Ansaugbereich als künstliche Mitralklappe (2) ausgebildet ist, wobei jeweils das anzusaugende Blut dem linken Vorhof (1) bestimmungsgemäß entnommen wird.

2. Herzunterstützungssystem nach Anspruch 1,
**dadurch gekennzeichnet, dass**
die Saugverbindung (4, 3, 2) aus einem Antriebsstutzen (4) der Pumpenanordnung und einem daran angeordneten mitral-apikalen Anschlussstück (3) besteht, wobei am pumpenfernen Ende eine künstliche Mitralklappe (2) angeordnet ist, wobei die künstliche Mitralklappe (2) im Bereich der natürlichen Mitralklappe der linken Herzkammer (7) bestimmungsgemäß anordenbar ist.

3. Herzunterstützungssystem nach Anspruch 1,
**dadurch gekennzeichnet, dass**
die Saugverbindung (4, 3) aus einem Antriebsstutzen (4) der Pumpenanordnung und einem daran angeordneten mitral-apikalen Anschlussstück (3) besteht, wobei am pumpenfernen Ende eine Mitralklappenringprothese angeordnet ist, wobei die Mitralklappenringprothese an einem Mitralklappenring bestimmungsgemäß von der linken Herzkammer (7) anordenbar ist.

4. Herzunterstützungssystem nach Anspruch 1, 2 oder 3,
**dadurch gekennzeichnet, dass**
der Ansaugbereich und die Saugverbindung (4, 3) innerhalb der linken Herzkammer (7) bestimmungsgemäß führbar ist.

5. Herzunterstützungssystem nach einem der Ansprüche 1 bis 4,
**dadurch gekennzeichnet, dass**
der Auslassbereich (6a) bestimmungsgemäß innerhalb der Aorta (9) vorzusehen ist.

6. Herzunterstützungssystem nach einem der vorangehenden Ansprüche,
**dadurch gekennzeichnet, dass**
bestimmungsgemäß eine Kanüle zwischen der linken Herzkammer (7) und dem rechten Vorhof vorzusehen ist.

7. Herzunterstützungssystem nach einem der vorangehenden Ansprüche,
**dadurch gekennzeichnet, dass**
die Saugverbindung (4, 3, 2) zumindest abschnittsweise eine wenigstens auf der Innenseite spiralförmig ausgebildete Wandung (3a) aufweist, wobei ein in Pumpenrichtung wirkender unterstützender Bluttransport (10) erfolgt.

8. Herzunterstützungssystem nach Anspruch 7,
**dadurch gekennzeichnet, dass**
die spiralförmig ausgebildete Wandung der Saugverbindung (3a) mit einer geometrische Strukturierung, insbesondere durch Erhebungen, Bahnen, Leitkanäle, Leitwandungen, Einkerbungen und/oder Ausbuchtungen der Innenwand, eine Beschleunigung des Blutes in Blutrichtung (10) unterstützt.

9. Herzunterstützungssystem nach einem der vorangehenden Ansprüche,
**dadurch gekennzeichnet, dass**
die Saugverbindung (4, 3, 2) einteilig ausgebildet ist.

10. Herzunterstützungssystem nach einem der vorangehenden Ansprüche,
**dadurch gekennzeichnet, dass**
die Saugverbindung (4, 3, 2) eine Sensorik (11) zur Erfassung der Strömungsgeschwindigkeit, Strömungsbeschleunigung und/oder des Blutdurchflusses aufweist, wobei die Sensorik (11) mit der Pumpanordnung (5) zum Transport des Blutes verbindbar ist.

11. Herzunterstützungssystem nach Anspruch 10,
**dadurch gekennzeichnet, dass**
die Pumpanordnung (5) zum Transport des Blutes eine Regeleinheit (5a) aufweist, die mit der Sensorik (11) verbindbar ist.

## Claims

1. A cardiac support system comprising
- an intake region for the intake of blood,
- an outlet region (6a) for the issuance of the drawn-in blood,
- a pump arrangement (5) for transporting the blood from the intake region to the outlet region (6a),
- a pump-aorta connection (6) able to be passed exterior to the left ventricle (7), and
- a suction connection (4) from the intake region to the pump,
wherein some of the cardiac function is assumed by the cardiac support system, wherein
the intake region is arranged directly after an artificial mitral valve (2)
or
the intake region is in the form of an artificial mitral valve (2),
wherein in each case the blood to be drawn in is removed from the left atrium (1) as intended.

2. A cardiac support system according to claim 1,
**characterised in that**
the suction connection (4, 3, 2) is made up of a drive connection piece (4) of the pump arrangement and a mitral-apical connector (3) arranged thereon, wherein an artificial mitral valve (2) is arranged at the end remote from the pump, wherein the artificial mitral valve (2) can be arranged in the region of the natural mitral valve of the left ventricle (7) as intended.

3. A cardiac support system according to claim 1, **characterised in that** the suction connection (4, 3) is made up of a drive connection piece (4) of the pump arrangement and a mitral-apical connector (3) arranged thereon, wherein a mitral valve ring prosthesis is arranged at the end remote from the pump, wherein the mitral valve ring prosthesis can be arranged on a mitral valve ring of the left ventricle (7) as intended.

4. A cardiac support system according to claim 1, 2 or 3,
**characterised in that**
the intake region and the suction connection (4, 3) are able to be passed within the left ventricle (7) as intended.

5. A cardiac support system according to any one of claims 1 to 4,
**characterised in that**
the outlet region (6a) is to be provided within the aorta (9) as intended.

6. A cardiac support system according to any one of the preceding claims,
**characterised in that**
a cannula is to be provided between the left ventricle (7) and the right atrium as intended.

7. A cardiac support system according to any one of the preceding claims,
**characterised in that**
at least portions of the suction connection (4, 3, 2) have a wall (3a) which is spiral-shaped on the inside at least, wherein there takes place a supporting blood transportation (10) acting in the pumping direction.

8. A cardiac support system according to claim 7,
**characterised in that** the spiral-shaped wall of the suction connection (3a) supports acceleration of the blood in the blood direction (10) with geometric structuring, in particular by protuberances, tracks, guide channels, guide walls, indentations and/or bulging of the inner wall.

9. A cardiac support system according to any one of the preceding claims,
**characterised in that**
the suction connection (4, 3, 2) is one-piece in design.

10. A cardiac support system according to any one of the preceding claims,
**characterised in that**
the suction connection (4, 3, 2) has a sensor system (11) for detecting the flow speed, the flow acceleration and/or the blood throughflow, wherein the sensor system (11) is connectable to the pump arrangement (5) for transportation of the blood.

11. A cardiac support system according to claim 10,
**characterised in that**
the pump arrangement (5) for blood transportation has a regulating unit (5a) connectable to the sensor system (11).

## Revendications

1. Système d'assistance cardiaque comportant
- une zone d'aspiration pour aspirer du sang,
- une zone de sortie (6a) pour distribuer le sang aspiré,
- un dispositif de pompage (5) pour transporter le sang de la zone d'aspiration vers la zone de sortie (6a),
- une connexion pompe-aorte (6) qui peut être placée à l'extérieur du ventricule gauche (7), et
- un raccord d'aspiration (4) de la zone d'aspiration vers la pompe,
certaines fonctions cardiaques étant assurées par le système d'assistance cardiaque, la zone d'aspiration étant disposée directement à la suite d'une valve mitrale artificielle (2)
ou
la zone d'aspiration étant réalisée comme une valve mitrale artificielle (2),
le sang à aspirer étant prélevé de façon conforme de l'oreillette gauche (1).

2. Système d'assistance cardiaque selon la revendication 1,
**caractérisé en ce que**
le raccord d'aspiration (4, 3, 2) est constitué d'une prise d'entraînement (4) du dispositif de pompage et d'un élément de raccordement mitral-apical (3) disposé sur celle-ci, une valve mitrale artificielle (2) étant disposée à l'extrémité éloignée de la pompe, la valve mitrale artificielle (2) pouvant être disposée de façon conforme dans la zone de la valve mitrale naturelle du ventricule gauche (7).

3. Système d'assistance cardiaque selon la revendication 1,
**caractérisé en ce que**
le raccord d'aspiration (4, 3) se compose d'une prise d'entraînement (4) du dispositif de pompage et d'un élément de raccordement mitral-apical (3) disposé sur celle-ci, une prothèse à anneau de valve mitrale étant disposée à l'extrémité éloignée de la pompe, la prothèse à anneau de valve mitrale pouvant être disposée de façon conforme sur un anneau de valve mitrale du ventricule gauche (7).

4. Système d'assistance cardiaque selon la revendication 1, 2 ou 3,
**caractérisé en ce que**
la zone d'aspiration et le raccord d'aspiration (4, 3) peuvent être placés de façon conforme à l'intérieur du ventricule gauche (7).

5. Système d'assistance cardiaque selon l'une quelconque des revendications 1 à 4,
**caractérisé en ce que**
la zone de sortie (6a) doit être prévue de façon conforme à l'intérieur de l'aorte (9).

6. Système d'assistance cardiaque selon l'une des revendications précédentes,
**caractérisé en ce qu'**
une canule doit être prévue de façon conforme entre le ventricule gauche (7) et l'oreillette droite.

7. Système d'assistance cardiaque selon l'une des revendications précédentes,
**caractérisé en ce que**
le raccord d'aspiration (4, 3, 2) présente au moins par sections une paroi (3a) formée au moins à l'intérieur en spirale, un transport de sang (10) d'assistance agissant dans le sens de la pompe se produisant.

8. Système d'assistance cardiaque selon la revendication 7,
**caractérisé en ce que**
la paroi formée en spirale du raccord d'aspiration (3a) avec une structure géométrique favorise une accélération du sang dans la direction de circulation du sang (10), en particulier par des reliefs, des chemins, des canaux de guidage, des parois de guidage, des entailles et/ou des renflements de la paroi interne.

9. Système d'assistance cardiaque selon l'une des revendications précédentes,
**caractérisé en ce que**
le raccord d'aspiration (4, 3, 2) est réalisé en une seule pièce.

10. Système d'assistance cardiaque selon l'une des revendications précédentes,
**caractérisé en ce que**
le raccord d'aspiration (4, 3, 2) est muni d'un système de capteurs (11) pour détecter la vitesse de l'écoulement, l'accélération de l'écoulement et/ou le flux sanguin, le système de capteurs (11) pouvant être connecté au dispositif de pompage (5) pour transporter le sang.

11. Système d'assistance cardiaque selon la revendication 10,
**caractérisé en ce que**
le dispositif de pompage (5) pour transporter le sang est muni d'une unité de commande (5a) qui peut être reliée au système de capteurs (11).
